# EUROPÄISCHE PATENTSCHRIFT

(11) **EP 0 978 260 B1**
(45) Veröffentlichungstag und Bekanntmachung des Hinweises auf die Patenterteilung: **19.11.2003**
(21) Anmeldenummer: 99114758.8
(22) Anmeldetag: 28.07.1999
(51) Int. Cl.: A61F 2/30

(54) **Zementfrei implantierbare Endoprothese aus Metall und Verfahren zu ihrer Herstellung**
Metal endoprothesis implantable without cement, and process for manufacturing it
Endoprothèse en métal implantable sans ciment et son procédé de fabrication

(30) Priorität: 03.08.1998 DE 29813821 U
(43) Veröffentlichungstag der Anmeldung: 09.02.2000
(73) Patentinhaber: ESKA Implants GmbH & Co., 23556 Lübeck (DE)
(72) Erfinder: Henssge, Ernst Joachim, Prof.Dr.med, 23562 Lübeck (DE); Broziat, Horst, 23554 Lübeck (DE)
(74) Vertreter: Fuchs Mehler Weiss & Fritzsche

(56) Entgegenhaltungen:
- EP-A- 0 257 222
- DE-A- 3 716 026
- DE-U- 29 501 042
- FR-A- 2 744 010
- US-A- 4 718 914
- US-A- 4 904 262
- US-A- 5 015 817

## Beschreibung

Die Erfindung betrifft eine zementfrei implantierbare Endoprothese aus Metall, die mehrere Ausnehmungen an ihrer Oberfläche enthält und aus mindestens einem Blechzuschnitt besteht, an dessen Rand die Ausnehmungen angeordnet sind, sowie ein Verfahren zur Herstellung einer derartigen Endoprothese.

Endoprothesen, welche als Ersatz menschlicher Knochen dienen und zu diesem Zweck in den menschlichen Körper implantiert werden können, sind in den vielfältigsten Ausgestaltungen und Herstellungsverfahren bekannt. Die bekannten Metallendoprothesen besitzen eine dreidimensionale komplexe Form und werden in diese Form geschmiedet, gegossen oder durch spanende Verfahren erzeugt. Verschiedene Formen von Ausnehmungen sind an der Oberfläche der Metallendoprothesen eingeformt, um ein Anwachsen des menschlichen Knochengewebes nach Implantation der Endoprothese zu ermöglichen, und um insbesondere eine formschlüssige Verbindung des Knochengewebes mit der Endoprothese an der Grenzfläche Knochen/Endoprothese zu verwirklichen. Die Herstellung derartiger bekannter Endoprothesen ist je nach Form, Verwendungszweck und Oberflächenstruktur oftmals recht aufwendig.

Aus der US-PS 4 595 393 ist eine Endoprothese, und zwar der Oberschenkelteil einer Hüftgelenkendoprothese bekannt, die aus dünnem Metallblech besteht, welches zu einer Hohlform geformt ist und den Schaft der Endoprothese bilden soll. Der hohle Schaft besitzt mehrere Ausnehmungen an seiner Oberfläche, in die das angrenzende Knochengewebe nach der Implantation einwachsen soll, um auf diese Weise Formschluss zwischen dem Knochengewebe und der Endoprothese zu erzielen und dadurch eine erhöhte Langzeitstabilität zu gewährleisten.

Aus der DE-A-37 16 026 ist eine gattungsgemäße Endoprothese bekannt, ebenso ein Verfahren zu deren Herstellung. Durch Biegen wird ein Metallblech in Form eines Gelenkstiles gebracht.

Aufgabe der Erfindung ist es, eine Endoprothese der eingangs genannten Art derart weiterzubilden, dass sie ein größeres dreidimensionales Knochenvolumen auszufüllen vermag.

Diese Aufgabe wird bei der Endoprothese der eingangs genannten Art erfindungsgemäß dadurch gelöst, dass mehrere Blechzuschnitte übereinander geschichtet als Laminat miteinander verbunden sind.

Ein Verfahren zum Herstellen einer zementfrei implantierbaren Endoprothese aus Metall, bei dem mindestens ein Blechzuschnitt aus einem Metallblech ausgeschnitten wird, und am Rand des Blechzuschnitts Ausnehmungen eingeformt werden, ist dadurch gekennzeichnet, dass mehrere Blechzuschnitte übereinander geschichtet und zu einem Laminat verbunden werden.

Die Vorteile der Erfindung liegen insbesondere darin, dass die Endoprothese aus einem oder mehreren Blechzuschnitten geformt ist, welcher an seinem Rand mehrere Ausnehmungen trägt, in welche das angrenzende Knochengewebe einwachsen kann.

Die Endoprothese wird als Laminat aus mehreren Blechzuschnitten verwirklicht, von denen mindestens ein Blechzuschnitt am Rand Ausnehmungen aufweist.

Bevorzugt sind die Blechzuschnitte bei ihrer Herstellung eben und werden dann, falls erforderlich, in die - von der Knochengestalt vorgegebene - räumlich gekrümmte Form geformt, die der Gestalt der zu ersetzenden Knochenpartie entspricht.

Gemäß einer bevorzugten Ausführungsform der Erfindung lässt sich ein Blechzuschnitt oder mehrere Blechzuschnitte zu einer Hohlform bilden, die einen Hohlraum einschließt. Mehrere Blechzuschnitte lassen sich zu diesem Zweck entsprechend krümmen und dann an geeigneten Stellen zusammenfügen, bevorzugt verschweißen.

Gemäß einer besonders bevorzugten Ausführungsform der Erfindung lassen sich Teile oder Abschnitte der Endoprothese aus massivem Metall herstellen, die fest mit den Blechzuschnitten verbunden sind. Die Blechzuschnitte werden bevorzugt an denjenigen Oberflächen, welche die Außenfläche der Endoprothese bilden, mit einer Oberflächenstruktur versehen, beispielsweise mit Ansätzen oder mit Nuten, besonders bevorzugt auch mit hinterschnittenen Nuten, welche das Einwachsen des Knochens in diese Strukturen ermöglichen und dadurch einen besonders festen Halt der Endoprothese nicht nur an ihrem Rand, sondern auch flächig an den Anlageflächen der Prothese ermöglichen.

Gemäß einer bevorzugten Ausführungsform der Erfindung verlaufen die Ausnehmungen vom Rand des Blechzuschnitts spaltförmig einwärts. Bevorzugt weisen einzelne oder mehrere benachbarte Ausnehmungen einen Hinterschnitt auf, in den das Knochengewebe allmählich einwachsen kann, um auf diese Weise einen besonders festen Formschluss zwischen Knochengewebe und Endoprothese zu gewährleisten.

Um die erfindungsgemäße Endoprothese herzustellen, wird mindestens ein Blechzuschnitt ausgeschnitten, und am Rand des Blechzuschnitts werden Ausnehmungen eingeformt. Vor oder nach dem Einarbeiten der Ausnehmungen werden die Blechzuschnitte in eine vorgegebene räumlich gekrümmte Form gebracht, die der gewünschten Prothesenform entspricht. Die Ausnehmungen besitzen bevorzugt Hinterschnitte. Mehrere Blechzuschnitte werden übereinander geschichtet und miteinander verbunden und bilden auf diese Weise eine dreidimensionale Endoprothese mit Vollquerschnitt.

Bevorzugt werden die Ausnehmungen am Rand der einzelnen Blechzuschnitte ganz oder teilweise im Hochdruck-Wasserstrahl-Verfahren oder in einem Laser-Schneidverfahren erzeugt. Zusätzlich lassen sich mittels des Hochdruck-Wasserstrahl-Verfahrens auch noch gewünschte Oberflächenstrukturen, beispielsweise hinterschnittene Nuten auf der Oberfläche der Blechzuschnitte anbringen.

Gemäß einer weiteren Ausführungsform der Erfindung ist mindestens ein Teil des Blechzuschnitts freigeschnitten und abkröpfbar; alternativ können auch Teile des Randes abgekröpft werden.

Vorteilhafte Weiterbildungen der Erfindung sind durch die Merkmale der Unteransprüche gekennzeichnet.

Im folgenden werden Ausführungsbeispiele der Erfindung anhand der Zeichnungen näher erläutert.

Es zeigen:
- Fig. 1: eine Aufsicht auf eine nicht erfindungsgemäße Endoprothese;
- Fig. 2: einen Schnitt längs der Linie II-II der Fig. 1;
- Fig. 3: eine der Fig. 2 entsprechende Darstellung, bei der jedoch erfindungsgemäß z.B. zwei Blechzuschnitte ein Schichtenlaminat bilden;
- Fig. 4: einen Querschnitt durch eine Hohlraum-Endoprothese; und
- Fig. 5 bis 8: Teilansichten verschiedener Ausführungsformen der Endoprothese.

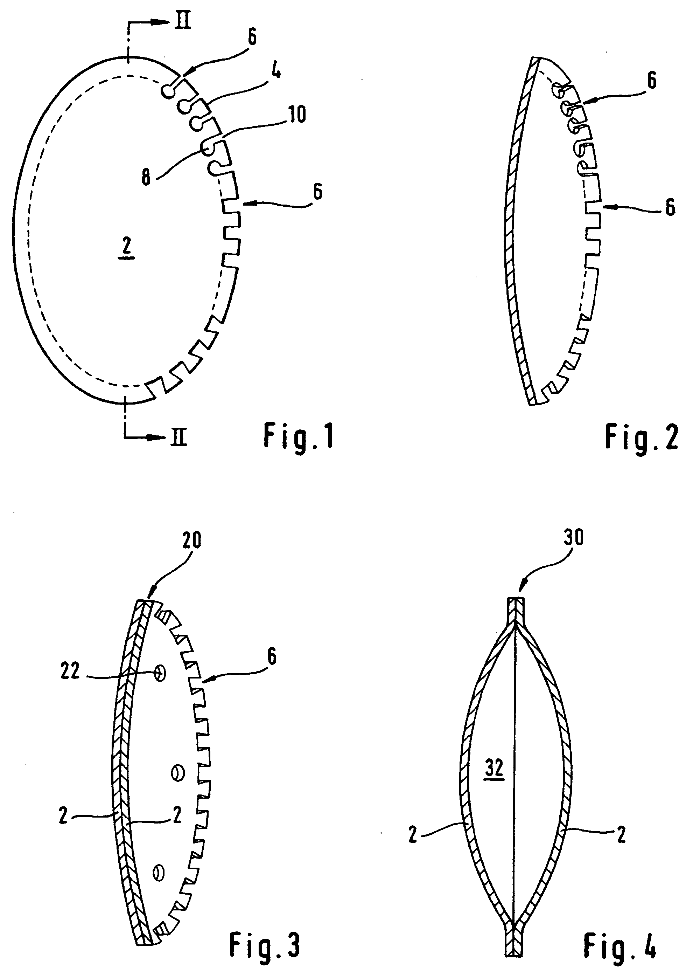

Die Fig. 1 und 2 zeigen eine Endoprothese aus Metall, die aus einem Blechzuschnitt 2 besteht, der entsprechend der zu ersetzenden Knochenpartie gekrümmt ist und überall dieselbe Dicke aufweist. Am Rand 4 der Endoprothese sind Ausnehmungen 6 eingeformt, die - gemäß den Fig. 1 und 2 - verschiedene Formen aufweisen können. Die Ausnehmungen 6 besitzen in der oberen dargestellten Ausführungsform je einen Hinterschnitt 8, der über einen verengten Spalt 10 am Rand 4 mündet. Alternative Ausführungsformen der Ausnehmungen 6 weisen - in Aufsicht - Rechteckform oder Trapezform auf.

Fig. 3 zeigt eine erfindungsgemäße Ausführungsform der Endoprothese, bei der zwei Blechzuschnitte 2 übereinandergeschichtet sind und ein Laminat bilden. Der Rand 4 der einzelnen Blechzuschnitte ist mit Ausnehmungen 6 versehen, die - in der dargestellten Ausführungsform - trapezförmig sich in das Zentrum der Blechzuschnitte 2 erstrecken. Alternativ ist es auch möglich, in dem Laminat einzelne Blechzuschnitte in den Schichtaufbau einzufügen, die am Rand keine Ausnehmungen 6 aufweisen. Vom Rand 4 beabstandet sind weitere Ausnehmungen 22, beispielsweise Bohrungen oder Vertiefungen, eingebracht. Alternativ können als Ausnehmungen in der Innenfläche auch Nuten vorgesehen werden, die Hinterschnitte besitzen.

Fig. 4 zeigt eine nicht erfindungsgemäße Ausführungsform 30 einer Endoprothese. Zwei Blechzuschnitte 2 sind bauchig konvex gekrümmt und werden an ihrem Rand 4 miteinander verbunden. Die Verbindung wird vorzugsweise mittels Laser-Schweißen durchgeführt. Am Rand sind außerdem noch Ausnehmungen (nicht dargestellt) eingebracht, die das formschlüssige Einwachsen des angrenzenden Knochengewebes ermöglichen sollen.

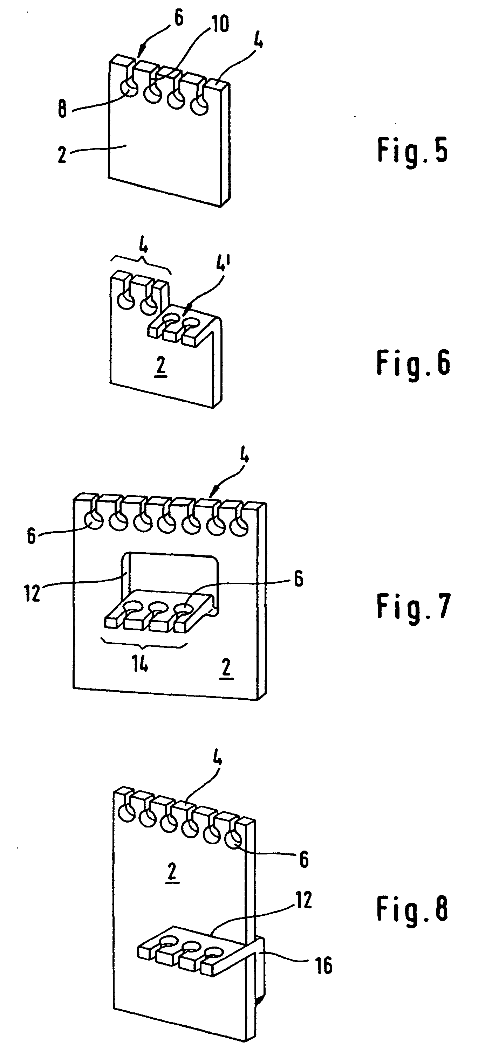

Die Fig. 5 bis 8 zeigen Teilansichten verschiedener nicht erfindungsgemäßer Ausführungsformen der Endoprothese. Fig. 5 zeigt den Rand 4 einer ebenen Endoprothese mit Ausnehmungen 6, die vom Rand schlitzartig (Schlitze 10) abgehen und Hinterschnitte 8 aufweisen. Fig. 6 zeigt eine der Fig. 5 entsprechende Endoprothese, deren Rand 4, 4' teilweise abgekröpft ist.

Die Fig. 7 und 8 zeigen Endoprothesen, bei denen ein Teil 14 mittels eines Trennschlitzes 12 freigeschnitten und dann abgekröpft und mit Randausnehmungen 6 versehen ist (Fig. 7), bzw. bei denen ein Schlitz 12 eingeschnitten ist, in den ein Blechteil 16 gestreckt und dort festgeschweißt ist.

## Patentansprüche

1. Zementfrei implantierbare Endoprothese aus Metall, mit mehreren Ausnehmungen an der Oberfläche, aus mindestens einem Blechzuschnitt (2), an dessen Rand (4) die Ausnehmungen (6) angeordnet sind, **dadurch gekennzeichnet, daß** mehrere Blechzuschnitte (2) übereinandergeschichtet als Laminat (20) miteinander verbunden sind.

2. Zementfrei implantierbare Endoprothese nach Anspruch 1, **dadurch gekennzeichnet, daß** mehrere Blechzuschnitte (2) miteinander verschweißt sind.

3. Endoprothese nach Anspruch 1, **dadurch gekennzeichnet, daß** die Blechzuschnitte eben sind.

4. Endoprothese nach Anspruch 1, **dadurch gekennzeichnet, daß** die Blechzuschnitte (2) eine räumlich gekrümmte Form besitzen.

5. Endoprothese nach einem der vorstehenden Ansprüche, **dadurch gekennzeichnet, daß** mehrere Blechzuschnitte (2) zu einer Hohlform zusammengefügt sind und einen Hohlraum (32) einschließen.

6. Endoprothese nach einem der vorstehenden Ansprüche, **dadurch gekennzeichnet, daß** Teile oder Abschnitte der Endoprothese als massive Metallteile ausgebildet sind, die fest mit den Blechzuschnitten (2) verbunden sind.

7. Endoprothese nach einem der vorstehenden Ansprüche, **dadurch gekennzeichnet, daß** mindestens ein Blechzuschnitt (2) vom Rand beabstandet weitere Ausnehmungen (12) enthält.

8. Endoprothese nach einem der vorstehenden Ansprüche, **dadurch gekennzeichnet, daß** die Blechzuschnitte (2) an denjenigen Oberflächen, welche die Außenfläche der Endoprothese bilden, eine vorgegebene Oberflächenstruktur aufweisen.

9. Endoprothese nach einem der vorstehenden Ansprüche, **dadurch gekennzeichnet, daß** die Ausnehmungen (6) vom Rand des Blechzuschnitts (2) spaltförmig einwärts in den Blechzuschnitt verlaufen.

10. Endoprothese nach einem der vorstehenden Ansprüche, **dadurch gekennzeichnet, daß** einzelne oder mehrere benachbarte Ausnehmungen (6) einen Hinterschnitt (8) aufweisen.

11. Endoprothese nach einem der vorstehenden Ansprüche, **dadurch gekennzeichnet, daß** ein Teil (4') des Randes (4) am Blechzuschnitt (2) gegenüber anderen Teilen des Randes (4) abgekröpft ist.

12. Endoprothese nach einem der vorstehenden Ansprüche, **dadurch gekennzeichnet, daß** Teile (14) des Blechzuschnitts (2) mittels eines Trennschlitzes (12) teilweise freigeschnitten und abgekröpft sind.

13. Endoprothese nach Anspruch 12, **dadurch gekennzeichnet, daß** die abgekröpften Teile ( 14) des Blechzuschnitts (2) am Rand Ausnehmungen (6) aufweisen.

14. Endoprothese nach einem der vorstehenden Ansprüche, **dadurch gekennzeichnet, daß** in den Blechzuschnitt (2) mindestens ein Schlitz (12) eingeschnitten ist, in den ein Blechteil (16) eingesetzt oder eingeschweißt ist.

15. Verfahren zum Herstellen einer Endoprothese nach einem der vorstehenden Ansprüche, bei dem mindestens ein Blechzuschnitt ausgeschnitten wird und am Rand des Blechzuschnitts Ausnehmungen eingeformt werden, **dadurch gekennzeichnet, daß** mehrere Blechzuschnitte übereinandergeschichtet und zu einem Laminat verbunden werden.

16. Verfahren nach Anspruch 15, **dadurch gekennzeichnet, daß** der/die Blechzuschnitte in eine vorgegebene, räumlich gekrümmte Form geformt werden.

17. Verfahren nach Anspruch 16, **dadurch gekennzeichnet, daß** vorgegebene Ausnehmungen mit Hinterschnitt gebildet werden.

18. Verfahren nach einem der Ansprüche 16 bis 17, **dadurch gekennzeichnet, daß** die Ausnehmungen ganz oder teilweise im Hochdruck-Wasserstrahl-Verfahren oder in einem Laser-Schneid-Verfahren erzeugt werden.

## Claims

1. Endoprosthesis made from metal which can be implanted without cement, having several recesses on the surface, made from at least one sheet metal blank (2), on the edge (4) of which the recesses (6) are arranged, **characterised in that** several sheet metal blanks (2) layered one above another are joined to one another as a laminate (20).

2. Endoprosthesis which can be implanted without cement according to claim 1, **characterised in that** several sheet metal blanks (2) are welded to one another.

3. Endoprosthesis according to claim 1, **characterised in that** the sheet metal blanks are flat.

4. Endoprosthesis according to claim 1, **characterised in that** the sheet metal blanks (2) have a spatially curved shape.

5. Endoprosthesis according to one of the above claims, **characterised in that** several sheet metal blanks (2) are assembled to form a hollow shape and enclose a cavity (32).

6. Endoprosthesis according to one of the above claims, **characterised in that** parts or sections of the endoprosthesis are designed as solid metal parts, which are joined firmly to the sheet metal blanks (2).

7. Endoprosthesis according to one of the above claims, **characterised in that** at least one sheet metal blank (2) at a distance from the edge contains further recesses (12).

8. Endoprosthesis according to one of the above claims, **characterised in that** the sheet metal blanks (2) have a preset surface structure at those surfaces which form the outer surface of the endoprosthesis.

9. Endoprosthesis according to one of the above claims, **characterised in that** the recesses (6) run from the edge of the sheet metal blank (2) like a slot inwards into the sheet metal blank.

10. Endoprosthesis according to one of the above claims, **characterised in that** individual or several adjacent recesses (6) have an undercut (8).

11. Endoprosthesis according to one of the above claims, **characterised in that** a part (4') of the edge (4) on the sheet metal blank (2) is offset with respect to other parts of the edge (4).

12. Endoprosthesis according to one of the above claims, **characterised in that** parts (14) of the sheet metal blank (2) are partly cut free and offset by means of a separating slot (12).

13. Endoprosthesis according to claim 12, **characterised in that** the offset parts (14) of the sheet metal blank (2) have recesses (6) on the edge.

14. Endoprosthesis according to one of the above claims, **characterised in that** at least one slot (12), into which a sheet metal part (16) is inserted or welded, is cut into the sheet metal blank (2).

15. Process for producing an endoprosthesis according to one of the above claims, in which at least one sheet metal blank is cut out and recesses are shaped on the edge of the sheet metal blank, **characterised in that** several sheet metal blanks are layered one above another and joined to form a laminate.

16. Process according to claim 15, **characterised in that** the sheet metal blank or blanks are shaped in a preset, spatially curved shape.

17. Process according to claim 16, **characterised in that** preset recesses with undercut are formed.

18. Process according to one of claims 16 to 17, **characterised in that** the recesses are produced wholly or partly by the high-pressure water-jet process or by a laser-cutting process.

## Revendications

1. Endoprothèse en métal implantable sans ciment, avec plusieurs évidements à sa surface, en au moins une découpe de tôle (2) au bord (4) de laquelle les évidements (6) sont disposés, **caractérisée en ce que** plusieurs découpes de tôle (2) superposées en couches sont assemblées entre elles en un laminé(20).

2. Endoprothèse en métal implantable sans ciment selon la revendication 1, **caractérisée en ce que** plusieurs découpes de tôle (2) sont soudées ensemble.

3. Endoprothèse selon la revendication 1, **caractérisée en ce que** les découpes de tôle sont planes.

4. Endoprothèse selon la revendication 1, **caractérisée en ce que** les découpes de tôle (2) ont une forme cintrée spatialement.

5. Endoprothèse selon l'une quelconque des revendications précédentes, **caractérisée en ce que** plusieurs découpes de tôle (2) sont assemblées en une forme creuse et enferment une cavité (32).

6. Endoprothèse selon l'une quelconque des revendications précédentes, **caractérisée en ce que** des pièces ou des sections de l'endoprothèse sont configurées comme pièces métalliques massives qui sont reliées à demeure aux découpes de tôle (2).

7. Endoprothèse selon l'une quelconque des revendications précédentes, **caractérisée en ce qu'**au moins une découpe de tôle (2) contient d'autres évidements (12) distants du bord.

8. Endoprothèse selon l'une quelconque des revendications précédentes, **caractérisée en ce que** les découpes de tôle (2) comportent une structure de surface prédéfinie aux surfaces qui forment la surface extérieure de l'endoprothèse.

9. Endoprothèse selon l'une quelconque des revendications précédentes, **caractérisée en ce que** les évidements (6) sont configurés en forme de fentes, qui s'étendent du bord de la découpe de tôle (2) vers l'intérieur dans la découpe de tôle.

10. Endoprothèse selon l'une quelconque des revendications précédentes, **caractérisée en ce que** certains évidements (6) ou plusieurs évidements (6) voisins comportent une contre-dépouille (8).

11. Endoprothèse selon l'une quelconque des revendications précédentes, **caractérisée en ce qu'**une partie (4') du bord (4) sur la découpe de tôle (2) est coudée par rapport aux autres parties du bord (4).

12. Endoprothèse selon l'une quelconque des revendications précédentes, **caractérisée en ce que** des parties (14) de la découpe de tôle (2) sont en partie dégagées au moyen d'une fente de séparation (12) et coudées.

13. Endoprothèse selon la revendication 12, **caractérisée en ce que** les parties coudées (14) de la découpe de tôle (2) comportent des évidements (6) au bord.

14. Endoprothèse selon l'une quelconque des revendications précédentes, **caractérisée en ce que**, dans la découpe de tôle (2), est taillée au moins une fente (12), dans laquelle une pièce de tôle (16) est introduite ou soudée.

15. Procédé de fabrication d'une endoprothèse selon l'une quelconque des revendications précédentes, selon lequel au moins une découpe de tôle est découpée et selon lequel des évidements sont pratiqués au bord de la découpe de tôle, **caractérisé en ce que** plusieurs découpes de tôle sont superposées en couches l'une au-dessus de l'autre et sont assemblées pour former un laminé.

16. Procédé selon la revendication 15, **caractérisé en ce que** la / les découpe(s) de tôle est / sont façonnée(s) en une forme cintrée spatialement.

17. Procédé selon la revendication 16, **caractérisé en ce que** des évidements prédéfinis à contre-dépouille sont formés.

18. Procédé selon l'une quelconque des revendications 16 à 17, **caractérisé en ce que** les évidements sont réalisés complètement ou partiellement selon le procédé par jet d'eau à haute pression ou selon un procédé de coupage au laser.
